(19) Europäisches Patentamt
European Patent Office
Office européen des brevets

(11) **EP 3 666 173 A1**

(12) **EUROPEAN PATENT APPLICATION**

(43) Date of publication:
17.06.2020 Bulletin 2020/25

(51) Int Cl.:
*A61B 5/00* (2006.01)  *A61B 5/04* (2006.01)
*A61B 5/0402* (2006.01)

(21) Application number: 18211254.0

(22) Date of filing: 10.12.2018

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Koninklijke Philips N.V.
5656 AG Eindhoven (NL)

(72) Inventor: FRANCK, Christoph Florian
5656 AE Eindhoven (NL)

(74) Representative: de Haan, Poul Erik et al
Philips International B.V.
Philips Intellectual Property & Standards
High Tech Campus 5
5656 AE Eindhoven (NL)

(54) **FILTERING UNIT FOR ELECTROCARDIOGRAPHY APPLICATIONS**

(57) The invention relates to a filtering unit (100) for compensating baseline drift in electrocardiographic real-time applications, which comprises a finite impulse response filter unit (106) configured to generate and provide a filtered digital electrocardiographic signal, and having an impulse response h[n] consisting of a finite sequence of between 900 and 160000 consecutive Kronecker delta pulses $\delta[n]$. The impulse response is formed by at least three consecutive sets of consecutive scaled Kronecker delta pulses, all scaled Kronecker delta pulses within a respective set having a respective constant amplitude, wherein a modulus of the sum of the amplitudes of every Kronecker delta pulse pulses is smaller than 0.1. A number of the scaled Kronecker delta pulses of at least one of the sets of Kronecker delta pulses is equal to or higher than a minimum number of samples resulting from multiplying the sampling frequency by a time span of $10^{-2}$ seconds.

FIG. 2

EP 3 666 173 A1

**Description**

FIELD OF THE INVENTION

The invention relates to a filtering unit for an electrocardiographic device, to a digital signal processor, to an electrocardiograph monitoring device and to a defibrillator.

BACKGROUND OF THE INVENTION

Electrocardiographic (ECG) signals are typically subject to low frequency noise such as, for example, from respiration that occurs at a lower rate than the heart rate, thus resulting in baseline wander, also referred to as baseline drift. Such an effect can render the ECG waveform difficult to read, especially in a display device having multiple ECG waveforms presented simultaneously. Presently, there are filtering techniques in existence that aggressively remove the baseline drift, but which also result in a distorted portion of ECG waveform (e.g., the ST segment) and/or introduce a delay in the display presentation. If a filtering technique is aimed at minimizing the distortion or eliminating a delay, this typically comes at the price of not aggressively correcting the baseline.

Typically, a known method for removing signal offsets and baseline wander implies the use of a single-pole high-pass filter with a corner frequency or cut-off frequency of 0.05Hz. Due to the value of the corner frequency, also referred to as cut-off frequency, the filter may need several seconds to move the ECG signal into a predetermined display range, especially when starting with a large offset. Thus, such a filter is usually combined with hardware or software monitoring of a current ECG signal level and with a signal-modification unit for accelerating the settling behavior if the ECG signal is outside the display range for too long. This typically requires additional hardware or software code and internal states machines to control the necessary operations to reduce the settling behavior.

US 2014/0142395 A1 describes a filter apparatus having a signal input to which an input signal is applied which contains a useful component and a noise component, a fast signal path and a slow signal path arranged in parallel therewith. The fast signal path and the slow signal path are coupled to the signal input. The fast signal path contains a filter in order to prompt fast filtering of the input signal. The slow signal path contains a filter in order to prompt slow filtering of the input signal. An output of the slow signal path is coupled to the fast signal path by means of a signal line. A signal output which is coupled to the fast signal path has an output signal applied to it which essentially contains useful components of the input signal. Disclosed therein is a method and apparatus for real time display of filtered electrocardiogram data. Briefly stated, a delayed symmetrical finite impulse response filter (FIR) is implemented to produce a continuously adjusted DC level portion of a display region. A continuous scrolling display is thereby provided in which a first portion of the display features a more recent portion of the overall waveform data having a continuously level adjusted, partially corrected baseline. In addition, a second portion of the display features an earlier portion of the overall waveform data having a substantially corrected baseline that scrolls at constant amplitude. As a result of this technique, the ST segment of a displayed ECG waveform remains undistorted.

SUMMARY OF THE INVENTION

It is desirable to provide a filtering unit for rejecting unwanted low-frequency components of an electrocardiographic signal requiring less expenditure for correcting a baseline of the electrocardiographic signal.

According to a first aspect of the present invention a filtering unit for ECG device is presented. The filtering unit comprises a digital signal input interface for receiving a digital electrocardiographic signal sampled at a predetermined sampling frequency that is between 300 and 8000 Hz, and thus suitable for ECG applications. The filtering unit also comprises a storage unit that is connected to the digital signal input interface and configured to store a predetermined number of consecutive recent samples of the digital electrocardiographic signal. The filtering unit also includes a finite impulse response filter unit connected to the storage unit. The finite impulse response filter unit is configured to generate and provide a filtered digital electrocardiograph signal using the stored samples. The finite impulse response filter unit has an impulse response $h[n]$ consisting of a finite ordered sequence of consecutive scaled Kronecker delta pulses $\delta[n]$, wherein n is an order of a respective Kronecker delta pulse in the finite ordered sequence. The total number of consecutive Kronecker delta pulses of the impulse response is between 900 and 160000. The impulse response is formed by at least three consecutive sets of one or more consecutive scaled Kronecker delta pulses, all scaled Kronecker delta pulses within a respective set having a respective constant amplitude.

The constant amplitudes of the respective sets of scaled Kronecker delta pulses are chosen so that a modulus of the sum of the amplitude of every Kronecker delta pulse of the at least three sets of Kronecker delta pulses is smaller than 0.1. The filtering unit thus allows implementing a high-pass filtering unit for rejecting unwanted low-frequency components of an electrocardiographic signal or a band-pass filtering unit having a lower corner frequency for rejecting the unwanted low-frequency components of the electrocardiographic signal.

Further, a number of the scaled Kronecker delta pulses of at least one of the sets of Kronecker delta pulses is equal to or higher than a minimum number of samples resulting from multiplying the sampling frequency by a time span of $10^{-2}$ seconds. In the filtering unit of the first aspect of the invention, the storage unit is configured to store at least a number of the consecutive recent samples of the digital electrocardiographic signal that is equal to the number of Kronecker delta pulses of the impulse response. In a mathematical notation, the impulse response disclosed by the present invention can thus be represented as:

$$h[n] = \sum_{i=0}^{j} \sum_{k=k_i}^{k_i+l_i-1} a_i \delta[n-k]$$

wherein

$j$ is a positive integer $> 1$;

$k_i$ and $l_i$ are positive integers, expect $k_0$, which is zero;

$k_{i+1} = k_i + l_i$;

$a_i \in \mathbb{R}$ are real numbers corresponding to the respective constant amplitudes and wherein at least two values of $a_i$ are not equal to zero.

The requirement $j > 1$ means that the impulse response in accordance with the present invention comprises at least three sets of scaled Kronecker delta pulses. The total number of sets of scaled Kronecker delta pulses is $j + 1$. Each of these sets of scaled Kronecker delta pulses has a number of consecutive Kronecker delta pulses given by $l_i$, and all Kronecker delta pulses of a given set are scaled by a common factor $a_i$, i.e., defining the respective constant amplitude for each of the $j + 1$ sets of Kronecker delta pulses. The total number $N$ of Kronecker delta pulses of the impulse response is given by:

$$N = \sum_{i=0}^{j} l_i$$

The fact that the modulus of the sum of the amplitudes of every Kronecker delta pulse of the at least three sets of Kronecker delta pulses is smaller than 0.1, ensures a minimum reduction of at least 90% of a DC component of an input signal, and thus suitably rejects unwanted low-frequency components of an electrocardiographic signal. In accordance with the notation introduced above, this requirement is expressed as:

$$\left| \sum_{i=0}^{j} a_i \cdot l_i \right| < 0.1$$

The filtering unit only requires one finite impulse response filter unit with the above-defined impulse response und thus requires less expenditure compared to known filtering units for an ECG device.

Further, having at least one of the sets of Kronecker delta pulses of the impulse response with a time span, determined in dependency on the sampling frequency, that is at least $10^{-2}$ seconds long further results in a reduction of the complexity of the filtering unit, as will be described further below. In accordance with the notation introduced above, this requirement is expressed as:

$\exists \, i \in (0,j)$ for which $(l_i > F_s \cdot 10^{-2})$

In the following, embodiments of the filtering unit of the first aspect of the invention will be described.

In a particularly preferred embodiment, , the impulse response of the finite impulse response filter unit is formed by a scaled, first Kronecker delta pulse $a_0\delta[n]$ having a predetermined first amplitude given by $a_0$ and forming the first set of scaled Kronecker delta pulses, and, following the first Kronecker delta pulse, at least two consecutive sets of further consecutive scaled Kronecker delta pulses, all scaled Kronecker delta pulses within a respective set having a respective constant amplitudes.

The impulse response of the finite impulse response filter is thus defined by:

$$h[n] = a_0 \delta[n] + \sum_{i=1}^{j} \sum_{k=k_i}^{k_i+l_i-1} a_i \delta[n-k]$$

wherein

$j > 1$;

$k_i$ and $l_i$ are positive integers;

$k_1 = 1$, and $k_{i+1} = k_i + l_i$;

$a_i \in \mathbb{R}$ and $a_0 \neq 0$, wherein $a_i$ are the values of the respective constant amplitudes.

The term $a_0\delta[n]$ corresponds to the first single scaled Kronecker delta pulse.

The total number $N$ of Kronecker delta pulses of the impulse response is given by:

$$N = 1 + \sum_{i=1}^{j} l_i$$

In this embodiment, the modulus of the sum of the first amplitude with the amplitudes of the scaled Kronecker delta pulses of the at least two consecutive sets of further consecutive scaled Kronecker delta pulses is smaller than 0.1.

In accordance with the notation introduced above, this requirement is expressed as:

$$\left| a_0 + \sum_{i=1}^{j} a_i \cdot l_i \right| < 0.1$$

Reducing the first set of scaled Kronecker deltas to a single Kronecker delta as it is the case in this embodiment, reduces the attenuation of higher-frequency components of the electrocardiographic signal, giving the finite-response filter unit a high-pass filter character.

In an alternative embodiment, wherein the first set of scaled Kronecker delta pulses is not a single scaled Kronecker delta pulse, a number of the scaled Kronecker delta pulses of the first set of Kronecker delta pulses is less than a maximum number of Kronecker delta pulses resulting from multiplying the sampling frequency by a time span of $8 \times 10^{-3}$ seconds. This embodiment provides a filtered digital electrocardiographic signal compliant with diagnostic-quality ECG.

Preferably, the predetermined amplitude of the first scaled Kronecker delta pulse has a first sign, for instance a positive sign, and the constant amplitudes $a_1$, $a_2$, ... $a_j$ of the at least two consecutive sets of further consecutive scaled Kronecker delta pulses have a sign opposite to the first sign, for instance a negative sign. In some embodiments, some but not all of the constant amplitudes $a_1$, $a_2$,... $a_j$ are zero.

The storage unit of the filtering unit of the first aspect of the invention has enough capacity to store at least the number of the consecutive recent samples of the digital electrocardiographic signal that is equal to the number of Kronecker delta pulses of the impulse response. In other words, the storage unit always stores the consecutive recent samples according to a first-in-first-out approach, corresponding to a moving window of a fixed number of recent samples, continuously discarding former samples that due to progress of time no longer count to the defined number recent samples.

Thus, technically, it is advantageous to use a low sampling frequency in order to minimize memory requirements. In a preferred embodiment the sample frequency is between 300 and 1000 Hz, preferably between 500 and 700 Hz, preferably 500 Hz for diagnostic-quality filtered digital electrocardiographic signals. In another embodiment, a sample frequency higher than 1000 Hz and up to 8000 Hz is used. In all embodiments, sufficient storage capacity is available in the storage unit.

In a particular embodiment, the finite impulse response filter unit is implemented using non-programmable digital hardware comprising a multi-stage digital delay line having a number of series-connected single delay units equal to a total number of consecutive Kronecker delta pulses of the impulse response. Each output of a respective signal delay unit and an input of the first single delay unit is branched out and fed to a respective multiplying unit for multiplication with a corresponding scaling factor for obtaining a partial product. All obtained partial products are added at an addition unit for providing the filtered digital electrocardiographic signal. This corresponds to a causal finite impulse response (FIR) filter of order N which is characterized by having a transfer function:

$$H(z) = \sum_{n=0}^{N} h[n] \cdot z^{-n}$$

which corresponds with a Z-transform of the impulse response $h[n]$. Each of the series-connected single delay units corresponds to a $z^{-1}$ operator.

In a time-domain, the input ($x[n]$)-output ($y[n]$) relation of the FIR filter is defined by the discrete convolution of the input with the impulse response:

$$y[n] = \sum_{m=0}^{N} h[m]x[n-m]$$

The input $x[n]$ corresponds to the digital electrocardiographic signal sampled at the predetermined sampling frequency (Fs) and the output $y[n]$ corresponds to the filtered digital electrocardiographic signal.

In another embodiment, the filtering unit of the first aspect of the present invention comprises a finite impulse response filter unit that includes a sparse FIR filter and an integrator. FIR filters are referred to as sparse filters when a significant part of their coefficients are equal to zero. This means that although the filter may have a high order and therefore storing of a relatively long history of past input samples is required (i.e., as many input samples as the number of Kronecker delta pulses of the impulse response), only a few of the past input samples are actually used in the calculation of each output value. The filtering unit of the first aspect is particularly suitable for implementation using this structure. The number of multiply and addition operations per output sample is minimized. Those delayed input samples that are multiplied by the difference of two consecutive amplitudes of equal value result in a value of zero and are not used by the calculation. Therefore, only a number of multiply-accumulate operations equal to the number of times the amplitude values of the consecutive Kronecker delta pulses varies is needed.

In a preferred embodiment, the finite impulse response filter unit comprises a processor having a storage device for storing the ordered sequence of consecutive scaled Kronecker delta pulses of the impulse response $h[n]$ and wherein the generation of the filtered digital electrocardiographic signal is performed based on a dedicated software code stored in the processor for calculating and providing, for every sample of the filtered digital electrocardiographic signal $y[n]$, the convolution of the digital electrocardiographic signal $x[n]$ with the impulse response $h[n]$:

$$y[n] = \sum_{m=0}^{N} h[m]x[n-m]$$

Typically, ECG signals are small AC signals, usually in the frequency band between 0.67 Hz and 150 Hz and having a voltage AC-component with a range of +/- 5mV. Due to the electrochemical behavior of the interface between adhesive electrodes and patient's skin, large DC components of up to several hundred mV may be present. It is advantageous to reduce the DC component of the ECG signal, preferably completely, to keep the ECG signal within a predetermined display range, which is typically, but not necessarily +/- 5mV.

In an embodiment of the filtering unit of the first aspect of the invention, the modulus of the sum of the amplitude of all Kronecker delta pulses of the impulse response is smaller than 0.01. This embodiment thus ensures a minimum reduction of at least 99% of a DC component of an input signal. In a preferred embodiment, the sum of the amplitude of every Kronecker delta pulse of the at least three sets of Kronecker delta pulses is equal to zero. Therefore, this preferred embodiment with a vanishing sum is advantageously configured to completely remove the DC component of the digital electrocardiographic signal.

Correspondingly, in another embodiment wherein the first set of Kronecker delta pulses is formed by a scaled first Kronecker delta pulse $a_0\delta[n]$, the modulus of the sum of the amplitude $a_0$ of the first Kronecker delta pulse and the amplitude of every further Kronecker delta pulse of the at least two consecutive sets of further consecutive scaled Kronecker delta pulses is smaller than 0.01, and in a particular embodiment, preferably zero.

With a sampling frequency between 300 and 8000 Hz and a number of consecutive Kronecker delta pulses of the impulse response being between 900 and 160000, the impulse response has a total time span based on the sampling frequency and defined as a quotient of the number of consecutive Kronecker delta pulses and the sampling frequency that is between 3 and 20 seconds long. In an embodiment, impulse response has a quotient of the number of Kronecker delta pulses and the sampling frequency that corresponds to a total time span of the impulse response between three and ten seconds, and, in another embodiment, more preferably between 3 and 5 seconds. Thus, the total number of scaled Kronecker delta pulses of the impulse response $N$ is between $Fs \times 3$ and $Fs \times 20$, between $Fs \times 3$ and $Fs \times 10$ and between $Fs \times 3$ and $Fs \times 5$ respectively.

The scientific publication "Recommendations for standardization and specifications on automated electrocardiography: bandwidth and digital signal processing. A report for health professionals by an ad hoc writing group of the Committee on Electrocardiography and Cardiac Electrophysiology of the Council on Clinical Cardiology, American Heart Association" by J.J. Bailey at al, Circulation, 1990; 81(2): 730-9 recommends restrictions on the behavior of high-pass or band-pass filters for diagnostic ECG purposes. The recommended restrictions are now part of current ECG standards like IEC 60601-2-25. The restrictions are in the form that after a defined test impulse, which is supposed to simulate the energy content of the largest R-wave the ECG monitor will encounter, the displacement of the signal from the baseline caused by the filter must not exceed a certain value, and the slope of the signal must also be lower than a given limit. Based on the requirements for diagnostic-quality ECG, as recommended by J.J. Bailey et al. and currently implemented by the IEC 60601-2-25 standard, the shortest possible time span of the impulse response of a filter that is still compliant is 3.15 seconds. The number of samples of such an impulse response depends on the sampling frequency and is $Fs \times 3.15$. The same standards also require a maximum recovery time of 5 seconds after the ECG monitoring equipment is subjected to defibrillator pulses and similar disturbances. The number of samples of such an impulse response depends on the sampling frequency and is $Fs \times 5$.

Thus, this preferred embodiment has a total number of scaled Kronecker delta pulses of the impulse response $N$ is between $3.15 \times Fs$ and $5 \times Fs$. In this particular advantageous embodiment of a filtering unit in accordance with the first aspect of the invention, that is compliant with the recommendations of J. J. Bailey et al, and thus also with current ECG standards like IEC 60601-2-25, the time span of the impulse response of the proposed filter is between 3.15 and 5 seconds.

One property of an ECG signal that is important for the diagnosis of pathological conditions like myocardial damage is whether a segment between an S-wave and a T-wave (called the S-T-segment) is shifted up or down compared to a voltage value just before a start of a given QRS complex. Shifts greater than 0.1 mV in either direction are clinically significant and would be interpreted as pathological by a cardiologist or by automated ECG analysis software. Thus, an excessive distortion is defined as a distortion resulting in a shift of the S-T- segment greater than 0.1 mV in either direction. A time span of the impulse response of 3.15 seconds results in the shortest settling time that still complies with the ECG standard in term of excessive distortion. However, impulse response time spans of more than 3.15 can be chosen to give the filtering unit other desirable properties, for example lower displacement of the S-T-segment, lower slopes, or lower ripple in the frequency band.

In another embodiment wherein the first set of Kronecker delta pulses is formed by the scaled first Kronecker delta pulse, a respective number of the further scaled Kronecker delta pulses of each of the at least two consecutive sets of further consecutive scaled Kronecker delta following the first Kronecker delta pulse is equal to or larger than a minimum number of the Kronecker delta pulses that equals a product of the sampling frequency and a time span of 0.1 second.

Thus, each of the at least two consecutive sets of further Kronecker delta pulses has a respective time span, determined as a quotient of a total number of Kronecker delta pulses of the impulse response and the sampling frequency that is at least 100 milliseconds.

In another embodiment, wherein preferably but not necessarily every set of further Kronecker delta pulses has a respective time span, that is at least 100 milliseconds, the impulse response of the finite impulse response filter unit comprises no more than fifty sets of Kronecker delta pulses. Preferably the finite impulse response filter unit comprises no more than ten sets of Kronecker delta pulses and more preferably the finite impulse response filter unit comprises no more than six sets of Kronecker delta pulses.

Increasing the number of sets of Kronecker delta pulses, or using sets of Kronecker delta pulses with shorter times span allows for a finer control over the frequency response of the finite impulse response filter unit at the cost of higher computational effort.

In some embodiments, the impulse response is formed by 3151 scaled Kronecker delta pulses. In one such embodiment, a scaled first Kronecker delta pulse has a predetermined first amplitude equal to 12000/12000. Following that first Kronecker delta pulse, the impulse response has a first set of 2850 consecutive Kronecker delta pulses with a constant amplitude of -4/12000. Following that first set of consecutive Kronecker delta pulses, the impulse response has a second set of 100 consecutive Kronecker delta pulses with a constant amplitude of -3/12000. Following that second set of consecutive Kronecker delta pulses, the impulse response has a third set of 100 consecutive Kronecker delta pulses with a constant amplitude of -2/12000. Following that third set of consecutive Kronecker delta pulses, the impulse response has a fourth set of 100 consecutive Kronecker delta pulses with a constant amplitude of -1/12000. For a digital electrocardiographic signal sampled at a predetermined sampling frequency ($F_s$) of 1000 Hz, the impulse response results in a total time span of 3.151 seconds which is the shortest impulse response that fulfills the slope and displacement requirements according to the recommendations of J. J. Bailey et al, stating that a 1 (mV*sec) impulse input should neither produce a displacement greater than 0.3 mV after the input nor a slope exceeding 1 mV/sec. This filtering unit results in a gain of 25% at a frequency of 0.22 Hz, which may cause amplification of undesired low-frequency interference by up to 25%. Thus, this particular embodiment is suitable when the impulse response is required to be as short as the possible within the recommendations and wherein the gain at low frequencies is not of particular concern.

In alternative embodiments, the impulse response is formed by 3570 scaled Kronecker delta pulses. In one such embodiment, the scaled first Kronecker delta pulse has a predetermined first amplitude equal to 12014/12014. Following that first Kronecker delta pulse, the impulse response has a first set of 1995 consecutive Kronecker delta pulses with a constant amplitude of -4/12014. Following that first set of consecutive Kronecker delta pulses, the impulse response has a second set of 1005 consecutive Kronecker delta pulses with a constant amplitude of -3/12014. Following that second set of consecutive Kronecker delta pulses, the impulse response has a third set of 450 consecutive Kronecker delta pulses with a constant amplitude of -2/12014. Following that third set of consecutive Kronecker delta pulses, the impulse response has a fourth set of 119 consecutive Kronecker delta pulses with a constant amplitude of -1/12014. For a digital electrocardiographic signal sampled at a predetermined sampling frequency (Fs) of 1000 Hz, the impulse response results in a total time span of 3.57 seconds. This embodiment of the filtering unit thus has a longer time span of the impulse response as the previous embodiment. However, this embodiment does fulfill a recommendation by Baileys et at, that states that the gain of low-frequency components should not exceed 20% at 0.22 Hz. Thus, this particular embodiment is suitable when the impulse response is required to be as short as the possible but wherein the gain at low frequencies is of particular concern.

In yet other alternative embodiments, the impulse response is formed by 4001 scaled Kronecker delta pulses. In one such embodiment, the scaled first Kronecker delta pulse has a predetermined first amplitude equal to 12075/12075. Following that first Kronecker delta pulse, the impulse response has a first set of 1400 consecutive Kronecker delta pulses with a constant amplitude of -4/12075. Following that first set of consecutive Kronecker delta pulses, the impulse response has a second set of 1400 consecutive Kronecker delta pulses with a constant amplitude of -3/12075. Following that second set of consecutive Kronecker delta pulses, the impulse response has a third set of 1075 consecutive Kronecker delta pulses with a constant amplitude of -2/12075. Following that third set of consecutive Kronecker delta pulses, the impulse response has a fourth set of 125 consecutive Kronecker delta pulses with a constant amplitude of -1/12075. For a digital electrocardiographic signal sampled at a predetermined sampling frequency (Fs) of 1000 Hz, the impulse response results in a total time span of 4.001 seconds. This embodiment of the filtering unit thus has a longer time span of the impulse response as the two previous exemplary embodiments. However, in this embodiment does fulfil the gain of low-frequency components remains below 15%. This shows a trade-off between the time span of the filter response and the gain at low frequencies, e.g., 0.22 Hz.

In an embodiment, wherein any one of the previously described exemplary impulse responses or other suitable impulses responses are implemented using a sparse form FIR filter followed by an integrator, the division by the respective common factor of the amplitudes (e.g., 12000, 12014 and 12075 in the exemplary embodiments described above) is advantageously performed after an integration step by the integrator. In this way, possible round-off errors are avoided before the integration step.

Typical finite impulse response (FIR) filters of order $N$, $N+1$ being the number of samples of the impulse response of said filter, require N multiply-accumulate operations per output sample of the filtered digital electrocardiographic signal. However, the filtering unit of the first aspect of the invention, which has finite impulse response filter unit with an impulse response having a first Kronecker delta pulse and then sets of scaled Kronecker delta pulses, wherein all scaled Kronecker delta pulses within a respective set have a respective constant amplitude, the computational effort will only depend on a number of times a value of the impulse response, i.e. the amplitude of the scaled Kronecker delta pulses, changes. Thus, for an impulse response value that changes four times, the determination of an output sample of the filtered digital electrocardiographic signal only requires four multiply-accumulate operations, regardless of the order of the FIR filter.

Diagnostic ECG imposes special requirements on filters, band-pass or high-pass, applied to the ECG wave. One property of the ECG wave that is important for the diagnosis of pathological conditions like myocardial damage is whether the segment between the S-wave and the T-wave (called the S-T-segment) is shifted up or down compared to the voltage just before the beginning of the QRS complex. Shifts greater than 0.1 mV in either direction are clinically significant and would be interpreted as pathological by a cardiologist or by automated ECG analysis software. High-pass filtering usually introduces a small shift in the S-T-Segment that is typically proportional to the size of the R-wave in the QRS complex. There are ways to remove the low-frequency contents of the signals without such a shift, but they have drawbacks like being incapable of being used in real-time (for example using forward-backward filtering), introducing latency, or being non-linear operations. The filtering unit of the present invention is suitable for filtering the ECG signal to remove the low-frequency contents while allowing its use in real-time applications.

According to a second aspect of the present invention, a digital signal processor is described. The digital signal processor comprises a filtering unit in accordance with the first aspect of the invention or of any of its embodiments. The digital signal processor further comprises an analog-to-digital converter configured to receive an analog electrocardiographic signal, to sample the analog electrocardiographic signal at the predetermined sampling frequency, and to provide to the digital signal input interface of the filtering unit the digital electrocardiographic signal sampled at a predetermined sampling frequency and thus formed by a sequence of sampled values of the analog electrocardiographic signal.

The digital signal processor of the second aspect thus shares the advantages of the filtering unit of the first aspect or of any of its embodiments.

According to a third aspect of the present invention, an electrocardiograph monitoring system is described. The electrocardiograph monitoring system comprises a digital signal processor according to the second aspect of the invention. It further comprises a signal acquisition unit configured to detect and provide, to the analog-to-digital converter of the signal processing unit analog electrocardiographic signals and a user output interface connected to the digital signal processor and configured to receive the filtered digital ECG signal and to provide a visually or acoustically perceivable output signal indicative thereof. The electrocardiograph monitoring system thus shares the advantages of the filtering unit of the first aspect of the invention or of any of its embodiments.

In an embodiment of the electrocardiograph monitoring system, the signal acquisition unit comprises a set of electrodes. Also, in an embodiment, the user output interface is a display unit.

In a particular embodiment, the signal acquisition unit and the analog-to-digital converter are part of a first device of the electrocardiographic monitoring system, which is configured to generate and provide the digital electrocardiographic signal formed by a sequence of sampled values of the analog electrocardiographic signal to the digital signal input interface of the filtering unit, which forms in turn part of a second device of the electrocardiographic monitoring system. The provision of the digital electrocardiographic signal is in a particular embodiment performed via a wired connection. In another embodiment the provision of the digital electrocardiographic signal is performed wirelessly in accordance with a predetermined wireless communication protocol.

According to a fourth aspect of the present invention, a defibrillator comprising an electrocardiograph monitoring system according to the third aspect is disclosed. The defibrillator of the fourth aspect also shares the advantages of the filtering unit of the first aspect of the invention or of any of its embodiments.

It shall be understood that the filtering unit of claim 1, the digital signal processor of claim 7, the electrocardiograph monitoring system of claim 8, and the defibrillator of claim 9, have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows a schematic block diagram of an embodiment of a filtering unit.
Fig. 2 shows a representation of an impulse response of a finite impulse response high-pass filter unit of an embodiment of a filtering unit.
Fig. 3A shows a schematic block diagram of a discrete-time FIR filter in direct form.
Fig. 3B shows a schematic block diagram of an implementation of a high-pass filter using a sparse FIR filter followed by an integrator.

Fig. 4A shows a schematic block diagram of an embodiment of an electrocardiograph monitoring system comprising a digital signal processor.

Fig. 4B shows a schematic block diagram of another embodiment of an electrocardiograph monitoring system comprising a digital signal processor.

Fig. 5A und 5B show voltage versus time diagrams of filtered ECG signals using a filtering unit in accordance with the invention and a typical high-pass filter with a corner frequency of 0.05Hz.

DETAILED DESCRIPTION OF EMBODIMENTS

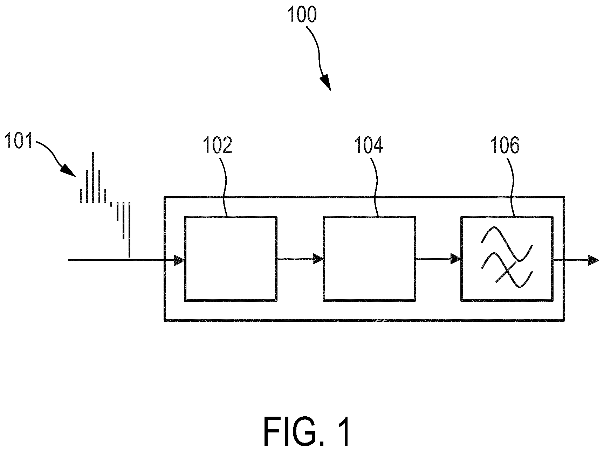

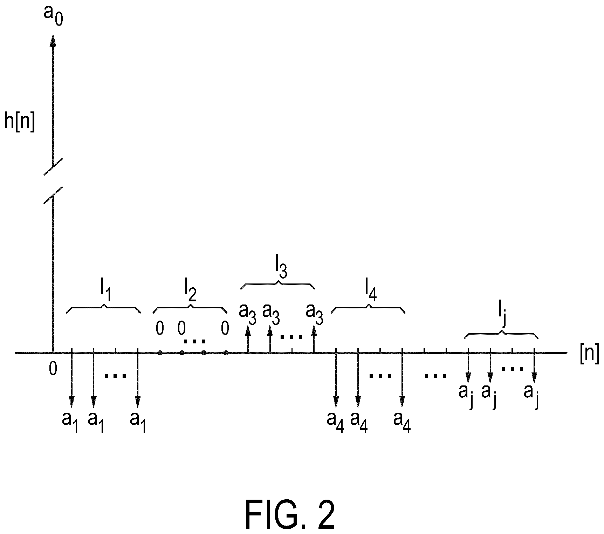

Fig. 1 shows a schematic block diagram of a filtering unit 100. The description of the filtering unit will be done with further reference to Fig. 2, which shows a representation of an impulse response of a finite impulse response high-pass filter unit of an embodiment of a filtering unit.

The filtering unit 100 comprises a digital signal input interface 102 for receiving a digital electrocardiographic 101 signal sampled at a predetermined sampling frequency $Fs$ between 300 and 8000 Hz. It also comprises a storage unit 104 that is connected to the digital signal input interface and configured to store a predetermined number of consecutive samples of the digital electrocardiographic signal.

The filtering unit 100 includes a finite impulse response filter unit 106 connected to the storage unit 104 and configured to generate and provide a filtered digital electrocardiographic signal using the stored samples, the finite impulse response filter unit having an impulse response $h[n]$. An exemplary impulse response is shown in Fig. 2. In general, a suitable impulse response consists of a finite ordered sequence of consecutive Kronecker delta pulses $\delta[n]$. The total number of consecutive Kronecker delta pulses of the impulse response is between 900 and 160000, and $n$ is an order of a respective Kronecker delta pulse in the finite ordered sequence.

The impulse response formed by at least three consecutive sets of consecutive scaled Kronecker delta pulses, all scaled Kronecker delta pulses within a respective set having a respective constant amplitude given by $a_0$, $a_1$, $a_2$, ... $a_j$. In the impulse response of Fig. 2, the first set of scaled Kronecker delta pulses is formed by a single scaled, first Kronecker delta pulse $a_0 \, \delta[n]$ having a predetermined first amplitude $a_0$.. Following the first Kronecker delta pulse, the exemplary impulse response shown in Fig. 2 comprises $j$ consecutive sets of further consecutive scaled Kronecker delta pulses, all scaled Kronecker delta pulses within a respective set having a respective constant amplitude of $a_1$,... $a_j$., wherein $j$ is a positive integer, in this case greater than five, but generally greater than 1. In this particular example the value of the constant $a_2$ is zero.

In the impulse response $h[n]$ of the finite impulse response high-pass filter unit 106, the modulus of the sum of the amplitude of each of the Kronecker delta pulse of the impulse response is smaller than 0.1. This relation can be described using the following expression:

$$\left| a_0 + \sum_{i=1}^{j} a_i \cdot l_i \right| < 0.1$$

Further, a number of the further scaled Kronecker delta pulses of at least one of the sets of Kronecker delta pulses ($l_1$, $l_2$, $l_3$, $l_4$...$l_j$) is equal to or higher than a minimum number of samples resulting from multiplying the sampling frequency $Fs$ by a time span of $10^{-2}$ seconds. For a sampling frequency of 300 Hz, at least one of the sets of Kronecker delta pulses is required to have at least three Kronecker delta pulses. For a sampling frequency of 8000 Hz, at least one of the sets of Kronecker delta pulses is required to have at least eighty Kronecker delta pulses.

Also, the storage unit 104 is configured to store at least a number of sample values equal to a total number of Kronecker delta pulses of the impulse response, which, as stated above, ranges between 900 and 160000.

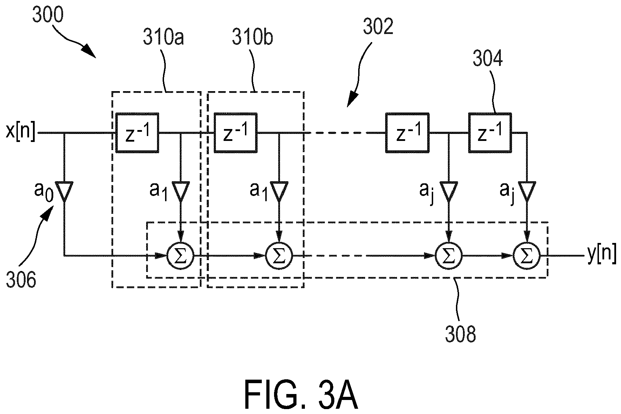

Fig. 3A shows a schematic block diagram of a discrete-time FIR filter in direct form. In a particular filtering unit, the finite impulse response high-pass filter unit is implemented using non-programmable digital hardware comprising a multi-stage digital delay line 302 having as many series-connected single delay units 304 as the total number of consecutive Kronecker delta pulses of the impulse response, wherein each output of a respective signal delay unit and as well as the input of the first single delay unit is branched out and fed to a respective multiplying unit 306 for multiplication with a corresponding scaling factor $a_i$ for obtaining a partial product and wherein all obtained partial products are added at an addition unit 308 for providing the filtered digital electrocardiographic signal. This corresponds to a causal finite impulse response (FIR) filter of order $N$ which is characterized by having a transfer function:

$$H(z) = \sum_{n=0}^{N} h\left[n\right] \cdot z^{-n}$$

which corresponds with a Z-transform of the impulse response $h[n]$. Each of the single delay units corresponds to a $z^{-1}$ operator.

In a time-domain, the input ($x[n]$)-output ($y[n]$) relation of the FIR filter is defined by the discrete convolution of the input with the impulse response:

$$y[n] = \sum_{m=0}^{N} h[m]x[n-m]$$

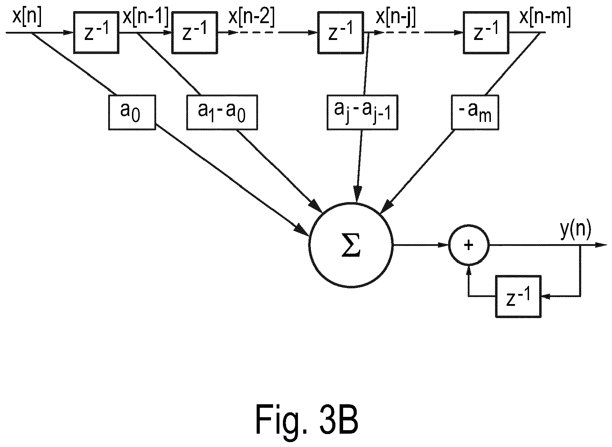

Typical FIR high-pass filters of order $N$, $N + 1$ being the number of samples of the impulse response of said high-pass filter, require $N + 1$ multiply-accumulate operations per output sample of the filtered digital electrocardiographic signal. However, in a filtering unit which has a finite impulse response high-pass filter unit with an impulse response such as the one described with reference to Fig. 2. i.e. an impulse response having a first Kronecker delta pulse and then $j$ sets of scaled Kronecker delta pulses, the computational effort will only depend on a number of times a value of the impulse response, i.e. the amplitude of the scaled Kronecker delta pulses, changes. Thus, for an impulse response value that changes, for instance, four times, the determination of an output sample of the filtered digital electrocardiographic signal $y[n]$ only requires four multiply-accumulate operations, regardless of the order of the FIR high-pass filter. This is shown in Fig. 3 based on the fact that the individual and consecutive multiply-accumulate units 310a and 310b, are identical.

In other filtering units (not shown), the finite impulse response high-pass filter unit comprises a processor having a storage device for storing the ordered sequence of consecutive scaled Kronecker delta pulses of the impulse response $h[n]$ and wherein the generation of the filtered digital electrocardiographic signal is performed based on a dedicated software code stored in the processor for calculating and providing, for every sample of the filtered digital electrocardiographic signal $y[n]$, the convolution of the digital electrocardiographic signal $x[n]$ with the impulse response $h[n]$.

Fig. 3B shows a schematic block diagram of an implementation of a high-pass filter using a sparse FIR filter followed by an integrator. FIR filters are referred to as sparse filters when a significant part of their coefficients are equal to zero. This means that although the filter may have a high order and therefore storing of a relatively long history of past input samples is required (i.e. as many input samples as the number of Kronecker delta pulse of the impulse response), only a few of the past input samples are actually used in the calculation of each output value. The fast-settling filtering unit design is particularly suitable for implementation using this structure. The number of multiply and addition operations per output sample is minimized. Those delayed input samples that are multiplied by the difference of two consecutive amplitudes, e.g. $a_j - a_{j-1}$, of equal value result in a value of zero and are not used by the calculation. Therefore, only a number of multiply-accumulate operations equal to the number of times the amplitude values of the consecutive Kronecker delta pulses varies is needed.

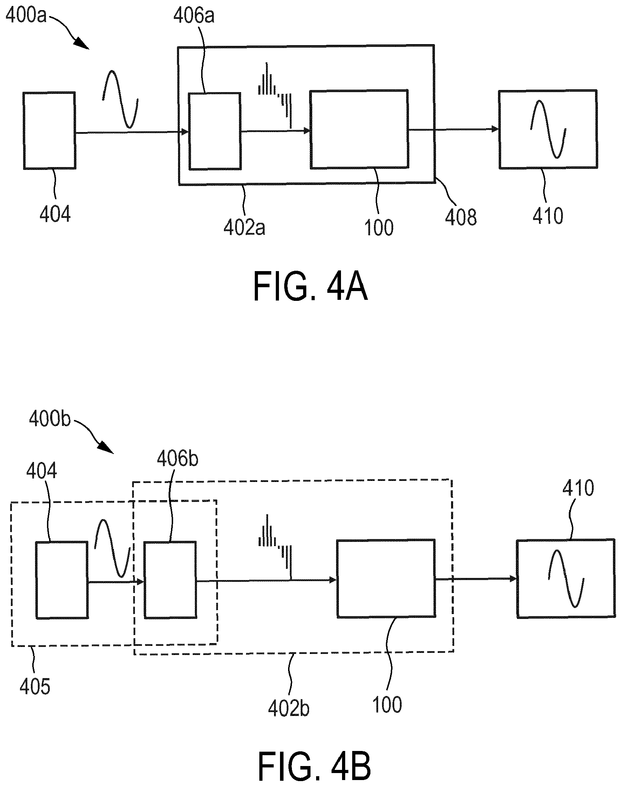

Figs. 4A and 4B show schematic block diagrams of two different electrocardiograph monitoring systems 400a, 400b comprising a digital signal processor 402a, 402b. Both electrocardiograph monitoring systems 400a and 400b comprise a filtering unit 100, as described with reference to Fig. 1. The different electrocardiograph monitoring systems 400a and 400b comprise a signal acquisition unit 404 configured to detect and provide, to a respective digital signal analog-to-digital converter 406a, 406b, the analog electrocardiographic signal: In the electrocardiograph monitoring system 400a, the analog-to-digital converter 406a and the filtering unit 100 are internal units of a digital signal processor device 408. In the electrocardiograph monitoring system 400b, the analog-to-digital converter 406b shares a housing signal acquisition unit 404 forming a signal acquisition device 405 configured to output a digital electrocardiographic signal sampled at the predetermined sampling frequency (Fs). This signal is transmitted in an exemplary electrocardiographic monitoring system via a wired connection. In an alternative electrocardiographic monitoring system, the transmission of the digital electrocardiographic (ECG) signal is performed wirelessly in accordance with a predetermined wireless communication protocol. Thus, in the exemplary electrocardiographic monitoring system 400b, the digital signal processor 402b is not necessarily embedded in a single device.

The electrocardiographic monitoring systems 400a and 400b further comprise a user output interface 410 connected to the digital signal processor and configured to receive the filtered digital ECG signal and to provide a perceivable output signal indicative thereof. The user output interface comprises in an exemplary electrocardiographic monitoring system a display device configured to visually represent the filtered digital electrocardiographic signal within predetermined signal threshold values. Alternatively, or additionally, the user output interface comprises a warning system, for instance optical or acoustical, configured to receive filtered digital ECG signal, to determine whether or not the received filtered ECG signal fulfils predetermined signal parameters and to provide a perceivable warning signal indicative of a non fulfilment of the signal parameters.

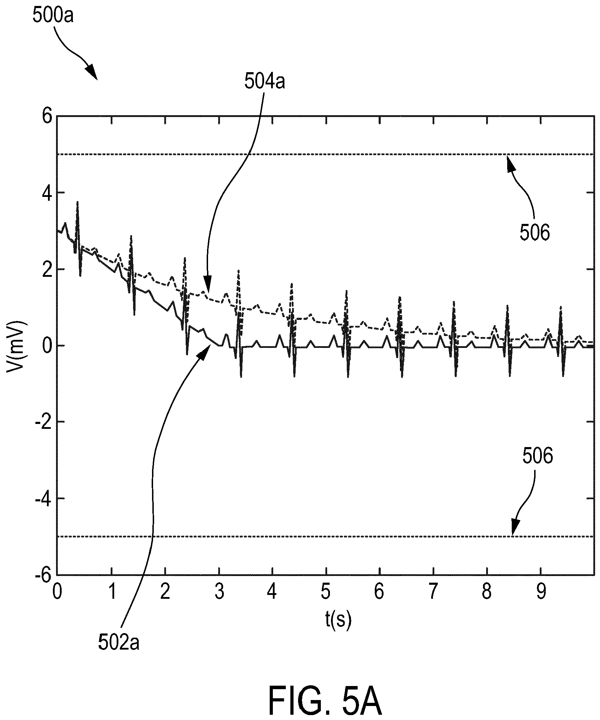

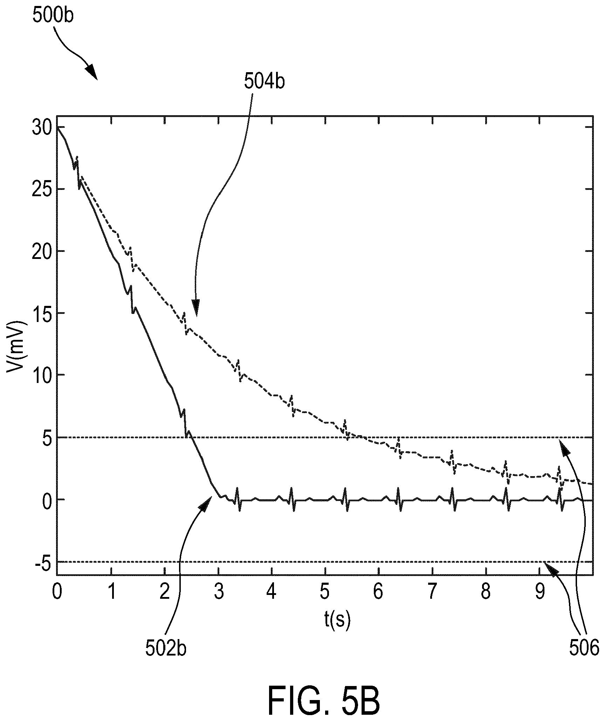

Figure 5a and 5b show a ECG voltage vs. time graphs 500a, 500b comparing a filtered digital ECG signal 502a, 502b obtained using a filtering unit with a filter response having a time span at the corresponding sampling frequency of 3.15 seconds and a filtered digital ECG signal 504a, 504b obtained with a commonly-used 0.05 Hz single-pole high-pass filter. Both filtered ECG signals are shown after being subject to an offset step in the ECG wave of 3mV in Fig. 5a and of 30 mV in Fig. 5b. Lines 506 represent the typical displayable voltage range for ECG applications, which is from +5mV to -5 mV. The digital filtered ECG signals 502a and 502b settle quickly and after a constant time corresponding to its impulse response time span. The ECG signals filtered using the single-pole 0.05 Hz high-pass settle slowly. Obtaining a similar response, suitable for real applications, requires a specific software configured to recognizes that the ECG signal is outside the displayable range and then to accelerate the settling of the single-pole filter by modifying the internal register contents or briefly switching to a higher corner frequency and then back to 0.05 Hz corner frequency. Other variations to the disclosed embodiments can be understood and effected by those skilled in the art in practicing the claimed invention, from a study of the drawings, the disclosure, and the appended claims.

A filtering unit according in accordance with the present the invention can be identified by the characteristics of its impulse response. In particular, use of the finite impulse response filter unit of the filtering unit described in the context of the embodiments of the present invention can be detected by feeding, as a test input signal, a test impulse representing a single Kronecker delta pulse. If use of the present invention is made, the response to this particular test input signal will consist of at least three consecutive sets of one or more consecutive scaled Kronecker delta pulses, all of the scaled Kronecker delta pulses within a respective set having a respective constant amplitude.

In summary the invention relates to a filtering unit for compensating baseline drift in electrocardiographic real-time applications, which comprises a finite impulse response filter unit configured to generate and provide a filtered digital electrocardiographic signal, and having an impulse response h[n] consisting of a finite sequence of between 900 and 160000 consecutive Kronecker delta pulses $\delta[n]$, the impulse response formed by at least three consecutive sets of consecutive scaled Kronecker delta pulses, all scaled Kronecker delta pulses within a respective set having a respective constant amplitude, wherein a modulus of the sum of the amplitudes of every Kronecker delta pulse pulses is smaller than 0.1, a number of the scaled Kronecker delta pulses of at least one of the sets of Kronecker delta pulses is equal to or higher than a minimum number of samples resulting from multiplying the sampling frequency by a time span of $10^{-2}$ seconds.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1. A filtering unit (100) for an electrocardiograph device, the filtering unit comprising:

   - a digital signal input interface (102) for receiving a digital electrocardiographic signal sampled at a predetermined sampling frequency ($F_s$) between 300 and 8000 Hz;
   - a storage unit (104) connected to the digital signal input interface and configured to store a predetermined number of consecutive recent samples of the digital electrocardiographic signal;
   - a finite impulse response filter unit (106) connected to the storage unit and configured to generate and provide a filtered digital electrocardiographic signal using the stored samples, the finite impulse response filter unit having an impulse response h[n] consisting of a finite ordered sequence of between 900 and 160000 consecutive Kronecker delta pulses $\delta[n]$, wherein n is an order of a respective Kronecker delta pulse in the finite ordered sequence, the impulse response formed by
   - at least three consecutive sets of one or more consecutive scaled Kronecker delta pulses, all of the scaled Kronecker delta pulses within a respective set having a respective constant amplitude, wherein
   - a modulus of the sum of the amplitude of all the Kronecker delta pulses of the impulse response is smaller than 0.1;
   - a number of the scaled Kronecker delta pulses of at least one of the sets of Kronecker delta pulses is equal to or higher than a minimum number of Kronecker delta pulses resulting from multiplying the sampling frequency by a time span of $10^{-2}$ seconds; and wherein
   - the storage unit is configured to store at least a number of the consecutive recent samples of the digital electrocardiographic signal that is equal to the number of Kronecker delta pulses of the impulse response.

2. The filtering unit (100) of claim 1, wherein the impulse response is formed by:

   - a scaled first Kronecker delta pulse $a_0\delta[n]$ having a predetermined first amplitude $a_0$ and forming a first set of the sets of scaled Kronecker delta pulses; and
   - following the first Kronecker delta pulse, at least two consecutive sets of further consecutive scaled Kronecker delta pulses, all of the scaled Kronecker delta pulses within a respective set having respective constant amplitudes.

3. The filtering unit of claim 1, wherein the modulus of the sum of the amplitude of all Kronecker delta pulses of the impulse response is zero.

4. The filtering unit of claim 1, wherein the impulse response has a quotient of the number of Kronecker delta pulses and the sampling frequency that corresponds to a total time span of the impulse response between three and ten seconds.

5. The filtering unit of claim 2, wherein a respective number of the further scaled Kronecker delta pulses of each of the at least two consecutive sets of further consecutive scaled Kronecker delta pulses following the first Kronecker delta pulse is equal to or larger than a minimum number of the Kronecker delta pulses that equals a product of the sampling frequency and a time span of 0.1 second.

6. The filtering unit of claim 1, wherein the impulse response of the finite impulse response filter unit comprises not more than fifty sets of Kronecker delta pulses.

7. A digital signal processor comprising:

> - a filtering unit in accordance with claim 1; and
> - an analog-to-digital converter configured:
> - to receive an analog electrocardiographic signal;
> - to sample the analog electrocardiographic signal at the predetermined sampling frequency; and
> - to provide to the digital signal input interface of the filtering unit the digital electrocardiographic signal formed by a sequence of sampled values of the analog electrocardiographic signal.

8. An electrocardiograph monitoring system comprising:

> - a digital signal processor according to claim 7;
> - a signal acquisition unit configured to detect and provide, to the analog-to-digital converter of the digital signal processor, the analog electrocardiographic signal; and
> - a user output interface connected to the digital signal processor and configured to receive the filtered digital electrocardiographic signal and to provide a visually or acoustically perceivable output signal indicative thereof.

9. A defibrillator comprising an electrocardiograph monitoring system according to claim 8.

FIG. 1

FIG. 2

FIG. 3A

Fig. 3B

FIG. 4A

FIG. 4B

FIG. 5A

FIG. 5B

Europäisches
Patentamt

European
Patent Office

Office européen
des brevets

# EUROPEAN SEARCH REPORT

Application Number

EP 18 21 1254

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2007/078354 A1 (HOLLAND ALEXANDER [US]) 5 April 2007 (2007-04-05) * paragraphs [0002], [0029], [0031] - [0033], [0039] * * figures 1, 4,6,9 * * claim 8 * | 1-9 | INV. A61B5/00 A61B5/04 A61B5/0402 |
| A | US 6 280 391 B1 (OLSON DANA J [US] ET AL) 28 August 2001 (2001-08-28) * figures 4,6 * * column 2, line 38 - line 65 * * column 3, line 52 - column 4, line 10 * | 1-9 | |
| A | LAKHWANI RINKY ET AL: "Design and Comparison of Digital Filters for Removal of Baseline Wandering from ECG Signal", 2013 5TH INTERNATIONAL CONFERENCE ON COMPUTATIONAL INTELLIGENCE AND COMMUNICATION NETWORKS, IEEE, 27 September 2013 (2013-09-27), pages 186-191, XP032525165, DOI: 10.1109/CICN.2013.48 [retrieved on 2013-11-07] * page 187 * | 1-9 | |
| A | FRANCISCO PERDIGON ROMERO ET AL: "Baseline wander removal methods for ECG signals: A comparative study", ARXIV.ORG, CORNELL UNIVERSITY LIBRARY, 201 OLIN LIBRARY CORNELL UNIVERSITY ITHACA, NY 14853, 30 July 2018 (2018-07-30), XP081022101, * page 2 * | 1-9 | |

TECHNICAL FIELDS SEARCHED (IPC)

A61B

The present search report has been drawn up for all claims

| Place of search | Date of completion of the search | Examiner |
|---|---|---|
| The Hague | 13 June 2019 | Vanderperren, Yves |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 21 1254

This annex lists the patent family members relating to the patent documents cited in the above-mentioned European search report.
The members are as contained in the European Patent Office EDP file on
The European Patent Office is in no way liable for these particulars which are merely given for the purpose of information.

13-06-2019

| Patent document cited in search report | | Publication date | Patent family member(s) | | Publication date |
|---|---|---|---|---|---|
| US 2007078354 | A1 | 05-04-2007 | CA | 2624760 A1 | 19-04-2007 |
| | | | JP | 2009509711 A | 12-03-2009 |
| | | | US | 2007078353 A1 | 05-04-2007 |
| | | | US | 2007078354 A1 | 05-04-2007 |
| US 6280391 | B1 | 28-08-2001 | US | 6280391 B1 | 28-08-2001 |
| | | | WO | 0046690 A1 | 10-08-2000 |

EPO FORM P0459

For more details about this annex : see Official Journal of the European Patent Office, No. 12/82

**EP 3 666 173 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140142395 A1

**Non-patent literature cited in the description**

- **J.J. BAILEY.** *Circulation,* 1990, vol. 81 (2), 730-9